# EUROPEAN PATENT APPLICATION

(11) **EP 4 835 522 A1**
(43) Date of publication of application: **04.11.2026**
(21) Application number: 26173873.6
(22) Date of filing: 22.04.2026
(51) Int. Cl.: G01V 3/28

(54) **SYSTEMS AND METHODS FOR MONITORING RESERVOIR FLUIDS WITH AN ANTENNA SYSTEM**

(30) Priority: 28.04.2025 US 202519191356
(71) Applicant: Services Pétroliers Schlumberger, 75007 Paris (FR); Schlumberger Technology B.V., 2514 JG The Hague (NL)
(72) Inventor: KARKI, Bishnu, Sugar Land, TX 77478 (US); HOMAN, Dean M., Sugar Land, TX 77478 (US); FREY, Mark, Sugar Land, TX 77478 (US); TAN, Kong Hauw Sarwa Bakti, Sugar Land, TX 77478 (US); LI, Albert, Sugar Land, TX 77478 (US); HAJJARI, Mohammad, Sugar Land, TX 77478 (US)
(74) Representative: Schlumberger Intellectual Property Department

(57) **Abstract**

Aspects of the present disclosure provide a transmitter for measuring resistivity of a geological formation. The transmitter including a transmitter axial coil disposed around a transmitter body, the transmitter body including a magnetic core, a first transmitter saddle coil disposed on an outer surface of the transmitter body including a central axis perpendicular to a central axis of the transmitter axial coil, and a second transmitter saddle coil disposed on the outer surface of the transmitter body including a central axis perpendicular to the central axis of the transmitter axial coil and perpendicular to the central axis of the first transmitter saddle coil.

## Description

### BACKGROUND

The present disclosure generally relates to the oil and gas industry. More specifically, the present disclosure relates to systems and methods for monitoring reservoir fluids in situ. Before and during wellbore operations where reservoir fluids are extracted, it is helpful to monitor and/or map reservoir fluids to improve reservoir production.

Accordingly, as there is a need for improved systems and methods monitoring reservoir fluids in situ.

### SUMMARY

Aspects of the present disclosure provide an antenna system for measuring resistivity of a geological formation. The antenna system including a transmitter and a receiver. The transmitter including, a transmitter axial coil disposed around a transmitter body, a first transmitter saddle coil disposed on an outer surface of the transmitter body including a central axis perpendicular to a central axis of the transmitter axial coil, and a second transmitter saddle coil disposed on the outer surface of the transmitter body including a central axis perpendicular to the central axis of the transmitter axial coil and perpendicular to the central axis of the first transmitter saddle coil. The receiver including a receiver axial coil disposed around a receiver body, a first receiver saddle coil disposed on an outer surface of the receiver body including a central axis perpendicular to the central axis of the axial receiver coil, and a second receiver saddle coil disposed on the outer surface of the receiver body including a central axis perpendicular to the central axis of the receiver axial coil and perpendicular to the central axis of the first receiver saddle coil.

Aspects of the present disclosure provide a transmitter for measuring resistivity of a geological formation. The transmitter including a transmitter axial coil disposed around a transmitter body, the transmitter body including a magnetic core, a first transmitter saddle coil disposed on an outer surface of the transmitter body including a central axis perpendicular to a central axis of the transmitter axial coil, and a second transmitter saddle coil disposed on the outer surface of the transmitter body including a central axis perpendicular to the central axis of the transmitter axial coil and perpendicular to the central axis of the first transmitter saddle coil.

Aspects of the present disclosure provide a method for measuring resistivity of reservoir fluids within a formation. The method including lowering a downhole tool into a formation of a wellbore, the downhole tool comprising a mandrel and a drill bit disposed at a distal end of the mandrel, lowering an antenna system configured to measure resistivity of a geological formation through a bore in the drill bit of the downhole tool, measuring a resistivity of the formation with the antenna system, and raising the downhole tool and the antenna system through the wellbore.

### BRIEF DESCRIPTION OF DRAWINGS

So that the manner in which the above-recited features of the disclosure can be understood in detail, a more particular description of the disclosure, briefly summarized above, may be had by reference to embodiments, some of which are illustrated in the appended drawings. It is to be noted, however, that the appended drawings illustrate only typical embodiments of this disclosure and are therefore not to be considered limiting of its scope, for the disclosure may admit to other equally effective embodiments. It is emphasized that, in accordance with the standard practice in the industry, various features are not drawn to scale. In fact, the dimensions of the various features may be arbitrarily increased or reduced for clarity of discussion.
Figure 1 illustrates a schematic view of an exemplary wellsite, according to one or more embodiments.
Figure 2 illustrates a schematic view of a drill bit and an antenna system for monitoring the reservoir fluids in a geological formation, according to one or more embodiments.
Figure 3A illustrates a side schematic view of the high frequency transmitter of the antenna system, according to one or more embodiments.
Figure 3B illustrates a side schematic view of the low frequency transmitter of the antenna system, according to one or more embodiments.
Figure 3C illustrates a side schematic view of the receiver of the antenna system, according to one or more embodiments.
Figure 4 illustrates an exemplary cross-section of the high frequency transmitter, the low frequency transmitter and the receiver, according to one or more embodiments.
Figure 5 illustrates a method for monitoring the resistivity of reservoir fluids within a formation, according to one or more embodiments.

To facilitate understanding, identical reference numerals have been used, where possible, to designate identical elements that are common to the figures. It is contemplated that elements disclosed in one embodiment may be beneficially utilized on other embodiments without specific recitation.

### DETAILED DESCRIPTION

Illustrative examples of the subject matter claimed below will now be disclosed. In the interest of clarity, not all features of an actual implementation are described in this specification. It will be appreciated which in the development of any such actual implementation, numerous implementation-specific decisions may be made to achieve the developers' specific goals, such as compliance with system-related and business-related constraints, which will vary from one implementation to another. Moreover, it will be appreciated which such a development effort, even if complex and time-consuming, would be a routine undertaking for those of ordinary skill in the art having the benefit of this disclosure.

Further, as used herein, the article "a" is intended to have its ordinary meaning in the patent arts, namely "one or more." Herein, the term "about" when applied to a value generally means within the tolerance range of the equipment used to produce the value, or in some examples, means plus or minus 10%, or plus or minus 5%, or plus or minus 1%, unless otherwise expressly specified. Further, herein the term "substantially" as used herein means a majority, or almost all, or all, or an amount with a range of about 51% to about 100%, for example. Moreover, examples herein are intended to be illustrative only and are presented for discussion purposes and not by way of limitation.

For the sake of brevity, all similar components have been given similar reference numbers with the same last two digits and a full description of such similar components may not be repeated herein. Similarly, for the sake of brevity, all like components have been given the same reference numbers, and a full description of such components may not be repeated herein.

Aspects of the present disclosure provide systems and methods for monitoring reservoir fluids with an antenna system. The antenna system includes a high frequency transmitter, a low frequency transmitter, and a receiver. The high frequency transmitter and low frequency transmitter generate and emit magnetic fields in three dimensions into the geological formation including the reservoir fluid. The receiver receives signals corresponding to the emitted magnetic fields corresponding to the tensor resistivity of the reservoir fluids in the geological formation. In one or more aspects, the antenna system can be deployed in a wellbore by being lowered through a drill bit at the end of a tool string and then tripped out of the wellbore thereby mapping the reservoir fluid in the geological formation through which the wellbore is drilled.

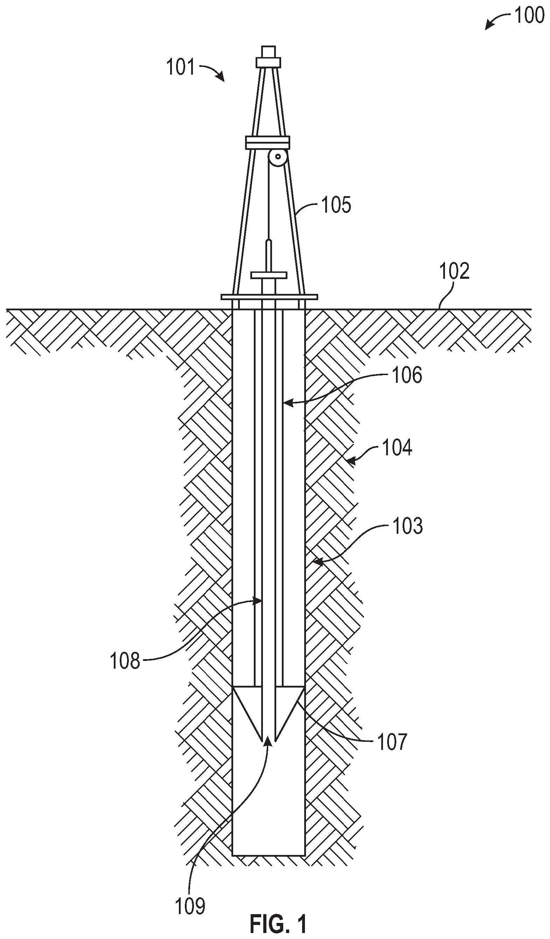

Figure 1 illustrates a schematic view of an exemplary wellsite 100. The wellsite 100 includes surface equipment 101 disposed on a surface 102 above a wellbore 103 formed in a geological formation 104. The geological formation 104 may contain reservoir fluid. Accordingly, the wellbore 103 may be utilized to extract the reservoir fluids disposed in the geological formation 104.

The surface equipment 101 includes a support system 105 for lowering and/or raising a tool string 106 into and out of the wellbore 103. In one or more embodiments, the tool string 106 may include an assembly of downhole tools coupled to one another and may include, for instance, one or more mandrels. In one or more embodiments, the tool string 106 may be used for drilling the wellbore 103. As illustrated, the tool string 106 may include a drill bit 107 disposed at the end of the tool string 106. The drill bit 107 is used to drill the wellbore 103 through the formation 104.

In one or more embodiments, the tool string 106 includes a bore therethrough 108 for conducting various wellbore operations with the tool string 106. For example, the tool string bore 108 may be used to transport drilling mud downhole, may be used to run wiring or hydraulics, or may be used for other mechanisms necessary to operate the tools of the tool string 106.

In one or more embodiments, the drill bit 107 also includes a bore 109. In one or more embodiments, the bore 109 of the drill bit 107 is fluidly coupled to the bore 108 of the tool string 106.

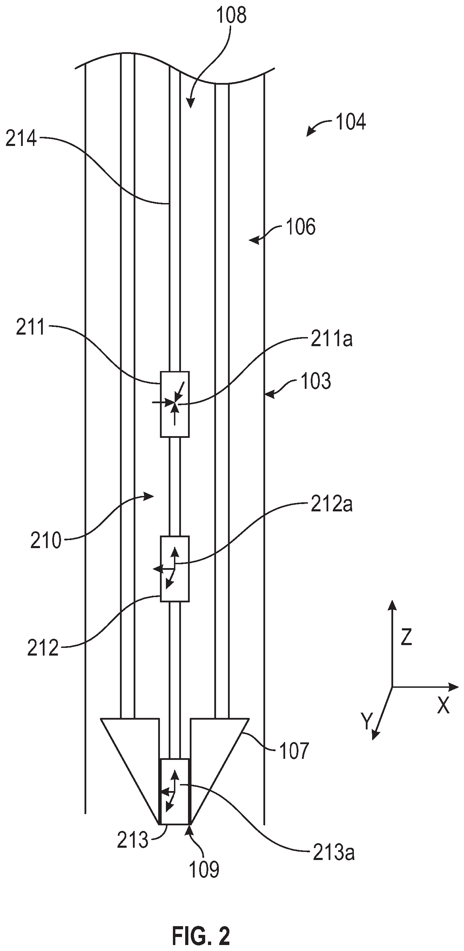

Figure 2 schematically illustrates the drill bit 107 and an antenna system 210 for monitoring the reservoir fluids in the geological formation 104. The antenna system 210 is configured to measure the resistivity (e.g., tensor resistivity) of the reservoir fluid within the geological formation 104 in three dimensions. In other words, the antenna system 210 can map the resistivity in the radial direction (or illustrated as the X and Y directions) as well as the axial direction (or illustrated as the Z direction). The mapping of the resistivity enhances the ability to monitor characteristics of the reservoir fluid, such as water infiltration. For example, the mapping of the resistivity allows for accounting in the variation of salinity for dynamic reservoir simulations.

The antenna system 210 is a triaxial antenna system 210 that includes a receiver 211 disposed above a high frequency transmitter 212 which is disposed above a low frequency transmitter 213. In one or more embodiments, the high frequency transmitter 212 is disposed from about 25 feet (ft) to about 75 ft from the receiver, such as less than or equal to 50 ft from the receiver 211, such as about 50 ft from the receiver 211. In one or more embodiments, the low frequency transmitter 213 is disposed from about 50ft to about 125 ft from the receiver 211, such as less than or equal to about 100 ft from the receiver 211, such as about 100 ft from the receiver 211. Without being limited by theory, the low frequency transmitter 213 being about 100 ft from the receiver 211 allows for approximately 100 ft in radial measurement. In one or more embodiments, the receiver 211, high frequency transmitter 212, and low frequency transmitter 213 are spaced apart to allow other tools to be disposed between them.

According to one mode of operation, the high frequency transmitter 212 generates and transmit magnetic fields in three dimensions (illustrated as arrows 212a) into the geological formation 104 and the low frequency transmitter 213 generates and transmits magnetic fields in three dimensions (illustrated as arrows 213a) into the geological formation 104. The high frequency transmitter 212 generating and transmitting a higher frequency and the low frequency transmitter 213 generating and transmitting a lower frequency. For example, the low frequency transmitter may operate at frequencies less than 20 kilohertz (kHz) and the high frequency transmitter may operate at frequencies between about 20 kHz and 100 kHz. Without being bound by theory, smaller frequency signals can travel further before being damped and thus can be further away from the receiver. However, the low frequency signals may have smaller signal to noise ratio than corresponding high frequency signals. On the other hand the high frequency transmitter may be closer to the receiver due to high attenuation for higher frequencies, but the signal to noise ratio may be larger relative to the lower frequencies.

The receiver 211 receives signals in three dimensions based on the transmitted magnetic fields (illustrated as arrows 211a). Those signals are indicative of the resistivity of the reservoir fluid residing in the geological formation 104.

As illustrated, according to one mode of operation, the antenna system 210 is coupled to a conveyance system 214 (*e.g.,* a narrower tool string). Accordingly, the various components of the antenna system 210 may be disposed along different axial positions of the conveyance system 214. The conveyance system 214 may physically *(i.e.,* mechanically couple) the various components of the antenna system 210, but may not necessarily electrically couple them. Electrical coupling is not necessary due to the electromagnetic signals being sent between the various components. The conveyance system 214 and antenna system 210 is sized to be lowered through the bore 108 in the tool string 106 and through the bore 109 in the drill bit 107. In one or more embodiments, the antenna system 210 has an outer diameter of 3.0 inches (in) or less, such as 2.5 in or less, such as 2.125 in or less, such as 2.125 in.

According to one mode of operation, the tool string 106 is lowered into the wellbore 103 and the drill bit 107 is used to drill the wellbore 103 through the formation 104. During or after drilling the wellbore 103, the antenna system 210 is lowered (such as by surface equipment 101 including support system 105) through the bore 108 of the tool string 106 and through the bore 109 of the drill bit 107. In one or more embodiments, the antenna system 210 may be lowered to the end of the drill bit 107. In one or more embodiments, the tool string 106 is tripped slightly upward from the bottom of the wellbore 103 to allow a portion of the antenna system 210 to protrude from the end of the drill bit 107.

The antenna system 210 maps (*e.g*., monitors or measures in three dimensions) the resistivity of the reservoir fluid within the formation 104. In one or more embodiments, the antenna system 210 maps as it is being lowered. In one or more embodiments, the antenna system 210 is tripped out of the wellbore 103 with the tool string 106 and maps as the tool string 106 and the antenna system 210 are tripped out. In one or more embodiments, the antenna system 210 is tripped out before the tool string 106 and maps while it is tripped out. In one or more embodiments, the antenna system 210 is tripped out after the tool string 106 is tripped out and it maps while it is tripped out. The antenna system 210 being lowered through the tool string 106 allows for the antenna 210 to be run into lateral wells (and/or lateral portions of a well) without the use of a tractor.

In one or more embodiments, the antenna system 210 is pre-installed in the tool string 106 and is thus simultaneously lowered with the tool string 106. While illustrated as being lowered through the tool string 106 and the drill bit 107, in one or more embodiments, the antenna system 210 is simply lowered by other methods, such as by a wireline in a distinct and separate operation from one using a tool string 106.

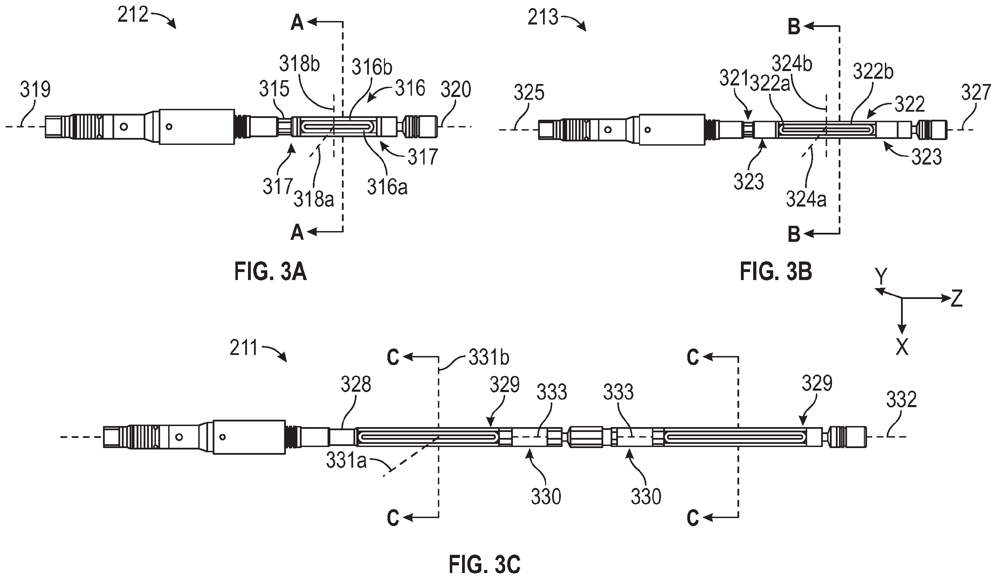

Figures 3A-3B illustrate schematic side views of the high frequency transmitter 212, the low frequency transmitter 213 and the receiver 211.

Figure 3A illustrates a schematic side view of the high frequency transmitter 212. The high frequency transmitter 212 includes a body 315, one or more saddle coils 316, and one or more axial coils 317. In one or more embodiments, the saddle coils 316 and the axial coils 317 are windings of wire, such as 28 gauge wire.

The saddle coils 316 are disposed on an outer surface of the body 315 and include a central axis 318 that is normal to the outer surface of the body 315 *(i.e.,* perpendicular to a longitudinal axis 319 of the body 315). The saddle coils 316 include X-direction saddle coils 316a and Y-direction saddle coils 316b. The X-direction saddle coils 316a include a central axis 318a in the X-direction. Accordingly, the X-direction saddle coils 316a generate and emit a magnetic field in the X-direction. The Y-direction saddle coils 316b are clocked 90° from the X-direction saddle coils 316a on the outer surface of the body 315. The Y-direction saddle coils 316b include a central axis 318b in the Y-direction. Accordingly, the Y-direction saddle coils 316b generate and emit a magnetic field in the Y-direction. In one or more embodiments, the high frequency transmitter 212 includes two X-direction saddle coils 316a and two Y-direction saddle coils 316b. In such embodiments, the two X-direction saddle coils 316a are clocked 180° from one another and the two Y-direction saddle coils 316b are clocked 180° from one another.

The axial coils 317 are wound around the outer surface of the body 315 and include a central axis 320 in the Z-direction *(i.e.,* parallel to the longitudinal axis 319 of the body 315). Accordingly, the axial coils generate and emit a magnetic field in the Z-direction.

In one or more embodiments, the axial coils 317 are wound around the outer surface of the body 315 at an end of the body 315 and adjacent to the portion of the body 315 including the saddle coils 316. In one or more embodiments, there are two axial coils 317 and they are each disposed at an end of the body 315 and the saddle coils 316 are disposed between the axial coils 317.

Figure 3B illustrates a schematic side view of the low frequency transmitter 213. The low frequency transmitter 213 includes the same or similar components to the high frequency transmitter 212. That is, the low frequency transmitter 213 includes a body 321, one or more saddle coils 322, and one or more axial coils 323.

The saddle coils 322 are disposed on an outer surface of the body 321 and include a central axis 324 that is normal to the outer surface of the body 321 (*i.e.,* perpendicular to a longitudinal axis 325 of the body 321). The saddle coils 322 include X-direction saddle coils 322a and Y-direction saddle coils 322b. The X-direction saddle coils 322a include a central axis 324a in the X-direction. Accordingly, the X-direction saddle coils 322a generate and emit a magnetic field in the X-direction. The Y-direction saddle coils 322b are clocked 90° from the X-direction saddle coils 322a on the outer surface of the body 321. The Y-direction saddle coils 322b include a central axis 324b in the Y-direction. Accordingly, the Y-direction saddle coils 322b generate and emit a magnetic field in the Y-direction. In one or more embodiments, the high frequency transmitter 212 includes two X-direction saddle coils 322a and two Y-direction saddle coils 322b. In such embodiments, the two X-direction saddle coils 322a are clocked 180° from one another and the two Y-direction saddle coils 322b are clocked 180° from one another.

The axial coils 323 are wound around the outer surface of the body 321 and include a central axis 327 in the Z-direction *(i.e.,* parallel to the longitudinal axis 325 of the body 321). Accordingly, the axial coils generate and emit a magnetic field in the Z-direction.

In one or more embodiments, the axial coils 323 are wound around the outer surface of the body 321 at an end of the body 321 and adjacent to the portion of the body 321 including the saddle coils 322. In one or more embodiments, there are two axial coils 323 and they are each disposed at an end of the body 321 and the saddle coils 322 are disposed between the axial coils 323.

In one or more embodiments, the coils *(i.e.* the saddle coils 316, 322 and the axial coils 317, 323) of the low frequency transmitter 213 include a higher number of turns than the coils *(i.e.* the saddle coils 316, 322 and the axial coils 317, 323) of the high frequency transmitter 212.

Figure 3C illustrates a schematic side view of the receiver 211. The receiver 211 includes a body 328, one or more saddle coils 329, and one or more axial coils 330. In one or more embodiments, the saddle coils 329 and the axial coils 330 are windings of wire, such as 28 gauge wire.

The saddle coils 329 are disposed on an outer surface of the body 328 and include a central axis 331 that is normal to the outer surface of the body 328 *(i.e.,* perpendicular to a longitudinal axis 332 of the body 328). The saddle coils 329 include X-direction saddle coils 329a and Y-direction saddle coils 329b. The X-direction saddle coils 329a include a central axis 331a in the X-direction. Accordingly, the X-direction saddle coils 329a generate and emit a magnetic field in the X-direction. The Y-direction saddle coils 329b are clocked 90° from the X-direction saddle coils 329a on the outer surface of the body 328. The Y-direction saddle coils 329b include a central axis 331b in the Y-direction. Accordingly, the Y-direction saddle coils 329b generate and emit a magnetic field in the Y-direction.

In one or more embodiments, the receiver 211 includes four X-direction saddle coils 329a and four Y-direction saddle coils 329b. In one or more embodiments, the four X-direction saddle coils 329a include two pairs of X-direction saddle coils 329a. Each pair of X-direction saddle coils 329a is located at the same axial location on the body 328 and are clocked 180° from one another. In one or more embodiments, the four Y-direction saddle coils 329b include two pairs of Y-direction saddle coils 329b. Each pair of Y-direction saddle coils 329b is located at the same axial location on the body 328 and are clocked 180° from one another. A pair of X-direction saddle coils 329a acts in series with the other pair of X-direction saddle coils 329a *(i.e.,* the X-direction saddle coils 329a at a first axial position on the body 328 act in series with the X-direction saddle coils 329a at a second axial position on the body 328). Similarly, a pair of Y-direction saddle coils 329b acts in series with the other pair of Y-direction saddle coils 329b *(i.e.,* the Y-direction saddle coils 329b at the first axial position on the body 328 act in series with the Y-direction saddle coils 329b at the second axial position on the body 328).

The axial coils 330 are wound around the outer surface of the body 328 and include a central axis 333 in the Z-direction *(i.e.,* parallel to the longitudinal axis 332 of the body 328). Accordingly, the axial coils generate and emit a magnetic field in the Z-direction. The axial coils 330 are located axially between the first pair of X-direction saddle coils 329a and the second pair of X-direction saddle coils 329a. The axial coils 330 are also located axially between the first pair of Y-direction saddle coils 329b and the second pair of Y-direction saddle coils 329b.

In one or more embodiments, the receiver 211 includes two axial coils 330 located at different axial locations on the body 328. In such embodiments, both axial coils 330 are located axially between the first pair of X-direction saddle coils 329a and the second pair of X-direction saddle coils 329a and between the first pair of Y-direction saddle coils 329b and the second pair of Y-direction saddle coils 329b.

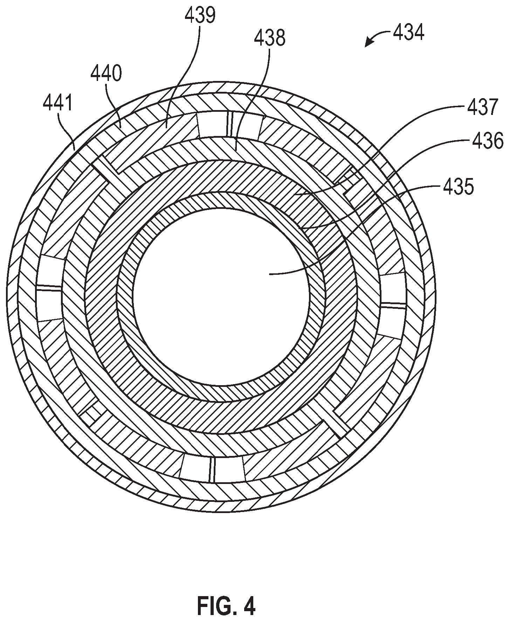

Figure 4 illustrates a cross section of the high frequency transmitter 212 (taken at section A-A), the low frequency transmitter 213 (taken at section B-B), and the receiver 211 (taken at sections C-C). In one or more embodiments, the bodies 315, 321, and 328 include the same cross-section at sections A-A, B-B, and C-C. Accordingly, for the purposes of Figure 4, the body shown is referred to herein as body 434.

The body 434 includes a core 435, a first layer 436 disposed around the core 435, a second layer 437 disposed around the first layer 436, a third layer 438 disposed around the second layer 437, the coils 439 are disposed around or on the outer surface of the third layer 438 (coils 439 refers equally to saddle coils 316, 322, 329 and axial coils 317, 323, and 330 as previously described), a fourth layer 440 disposed around the coils 439, and a fifth layer 441 disposed around the fourth layer 440.

In one or more embodiments, the core 435 includes a metallic material. The metallic material may be a steel, such as steel P550. In one or more embodiments, the first layer 436 includes an underwrap material, such as a Randolite underwrap material. In one or more embodiments, the second layer 437 includes a magnetic material. In one or more embodiments, the third layer 438 is a coil form, such as a Randolite coil form. In one or more embodiments, the fourth layer 440 includes an overlap material, such as a Randolite overlap material. In one or more embodiments, the fifth layer 441 includes an elastomer material, such as an elastomer molding disposed over the fourth layer 440.

Without being limited by theory, the magnetic properties of the body 434 assist in boosting the signals to compensate for reduction in signal due to a reduced diameter of the antenna system 210 as described above.

According to one mode of operation the transmitter coils of the antenna system 210 generate electromagnetic fields which pass through a geological formation in three dimensions. Due to the fluctuating field, voltage is induced in the receiver coil which is a function of area of the transmitter coil, area of the receiver coil, current in the transmitter coil, frequency of the signal generated by the transmitter, distance between the transmitter and receiver, and the resistivity of the formation. By knowing the transmitter-receiver characteristics, the resistivity of the formation can be determined. The triaxial nature of the antenna system 210 allows for full conductivity tensor, thereby giving information on formation resistivity anisotropy, dip, bed boundries, and other aspects of formation geometry.

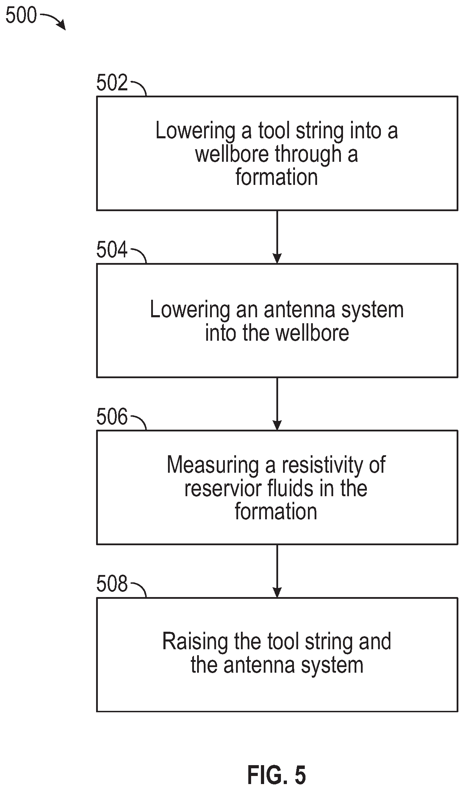

Figure 5 illustrates a method 500 for monitoring the resistivity of reservoir fluids within a formation (such as formation 104).

At operation 502, a tool string (such as tool string 106) is lowered into a wellbore (such as wellbore 103) within a geological formation (such as geological formation 104). In one or more embodiments, lowering the tool string includes drilling the wellbore. In such embodiments, a drill bit (such as drill bit 107) is used to drill the wellbore as the tool string is being lowered into the wellbore. In one or more embodiments, the wellbore is a lateral or partially-lateral wellbore.

At operation 504, an antenna system (such as antenna system 210) is lowered into the wellbore. In one or more embodiments, the antenna system is lowered through the tool string, such as through a bore (such as bore 108) of the tool string. The antenna system can be lowered through the tool string via a conveyance system. The antenna system can be lowered through a bore of the tool string. In one or more embodiments, the antenna system is lowered simultaneously with the tool string. In one or more such embodiments, the antenna system may be coupled to the tool string as the tool string is being lowered. In one or more embodiments, the antenna system is lowered after the tool string is lowered into the wellbore.

In one or more embodiments, the antenna system is lowered through a bore (such as bore 109) of the drill bit. The antenna system may be lowered to be flush with the tip *(i.e.,* distal end) of the drill bit. The antenna system may be lowered to protrude from the end of the drill bit. In some embodiments, the tool string may be raised slightly so that the antenna system may be sufficiently lowered so as to protrude from the tip of the drill bit.

At operation 506, the antenna system is used to measure the resistivity of reservoir fluids within the formation. The antenna system may include one or more transmitters (such as high frequency transmitter 212 and low frequency transmitter 213) generate and emit magnetic fields in three dimensions into the formation surrounding the wellbore. The antenna system may further include a receiver configured to receive signals corresponding the magnetic fields emitted by the one or more transmitters. The signals correlate to the resistivity of the reservoir fluid. Accordingly, the resistivity of the reservoir fluids within the formation can be mapped in three dimensions. In one or more embodiments, the antenna system measures the resistivity while the antenna system is lowered into the wellbore. In one or more embodiments, the antenna system measures the resistivity while the antenna system is raised from the wellbore (at operation 508).

At operation 508, the tool string and the antenna system are raised from the wellbore. In one or more embodiments, the tool string and the antenna system are raised simultaneously. In one or more embodiments, the tool string is raised before the antenna system is raised. In one or more embodiments, the antenna system is raised before the tool string is raised.

Examples in the present disclosure may also be directed to a non-transitory computer-readable medium storing computer-executable instructions and executable by one or more processors of the computer via which the computer-readable medium is accessed. A computer-readable media may be any available media which may be accessed by a computer. By way of example, such computer-readable media may comprise RAM, ROM, EEPROM, CD-ROM or other optical disk storage, magnetic disk storage or other magnetic storage devices, or any other medium which may be used to carry or store desired program code in the form of instructions or data structures and which may be accessed by a computer. Disk and disc, as used herein, includes compact disc (CD), laser disc, optical disc, digital versatile disc (DVD), floppy disk and Blu-ray^{®} disc where disks usually reproduce data magnetically, while discs reproduce data optically with lasers.

Note also that the software implemented aspects of the subject matter claimed below are usually encoded on some form of program storage medium or implemented over some type of transmission medium. The program storage medium is a non-transitory medium and may be magnetic (e.g., a floppy disk or a hard drive) or optical (e.g., a compact disk read only memory, or "CD ROM"), and may be read only or random access. Similarly, the transmission medium may be twisted wire pairs, coaxial cable, optical fiber, or some other suitable transmission medium known to the art. The claimed subject matter is not limited by these aspects of any given implementation.

### Example Aspects

Aspect 1: Aspects of the present disclosure provide an antenna system for measuring resistivity of a geological formation. The antenna system including a transmitter and a receiver. The transmitter including, a transmitter axial coil disposed around a transmitter body, a first transmitter saddle coil disposed on an outer surface of the transmitter body including a central axis perpendicular to a central axis of the transmitter axial coil, and a second transmitter saddle coil disposed on the outer surface of the transmitter body including a central axis perpendicular to the central axis of the transmitter axial coil and perpendicular to the central axis of the first transmitter saddle coil. The receiver including a receiver axial coil disposed around a receiver body, a first receiver saddle coil disposed on an outer surface of the receiver body including a central axis perpendicular to the central axis of the axial receiver coil, and a second receiver saddle coil disposed on the outer surface of the receiver body including a central axis perpendicular to the central axis of the receiver axial coil and perpendicular to the central axis of the first receiver saddle coil.

Aspect 2: The antenna system of Aspect 1, further comprising a second transmitter.

Aspect 3: The antenna system of Aspect 2, wherein the transmitter is disposed about 40 feet to about 60 feet from the receiver and the second transmitter is disposed about 80 feet to about 100 feet from the receiver.

Aspect 4: The antenna system of Aspects 2 or 3, wherein the transmitter is a high frequency transmitter and the second transmitter is a low frequency transmitter.

Aspect 5: The antenna system of any of Aspects 1-4, wherein the transmitter further includes a second transmitter axial coil, wherein the first transmitter saddle coil and the second transmitter saddle coil are axially disposed between the transmitter axial coil and the second transmitter axial coil.

Aspect 6: The antenna system of any of Aspects 1-5, wherein the transmitter body includes a magnetic core.

Aspect 7: The antenna system of any of Aspects 1-6, wherein the receiver body includes a magnetic core.

Aspect 8: The antenna system of any of Aspects 1-7, wherein the receiver further includes: a third receiver saddle coil disposed on the outer surface of the receiver body, wherein the third receiver saddle coil includes a central axis parallel to the central axis of the first receiver saddle coil, and wherein the first receiver saddle coil and the third receiver saddle coil operate in series; and a fourth receiver saddle coil disposed on the outer surface of the receiver body, wherein the fourth receiver saddle coil includes a central axis parallel to the central axis of the second receiver saddle coil, and wherein the second receiver saddle coil and the fourth receiver saddle coil operate in series.

Aspect 9: The antenna system of Aspect 8, wherein the receiver axial coil is axially disposed between the first receiver saddle coil and the third receiver saddle coil and between the second receiver saddle coil and the fourth receiver saddle coil.

Aspect 10: The antenna system of any of Aspects 1-9, wherein the transmitter further includes: a third transmitter saddle coil disposed on the outer surface of the transmitter body including a central axis parallel to the central axis of the first transmitter saddle coil; and a fourth transmitter saddle coil disposed on the outer surface of the transmitter body including a central axis parallel to the central axis of the second transmitter saddle coil.

Aspect 11: Aspects of the present disclosure provide a transmitter for measuring resistivity of a geological formation. The transmitter including a transmitter axial coil disposed around a transmitter body, the transmitter body including a magnetic core, a first transmitter saddle coil disposed on an outer surface of the transmitter body including a central axis perpendicular to a central axis of the transmitter axial coil, and a second transmitter saddle coil disposed on the outer surface of the transmitter body including a central axis perpendicular to the central axis of the transmitter axial coil and perpendicular to the central axis of the first transmitter saddle coil.

Aspect 12: The transmitter of Aspect 11, further comprising a second axial coil, wherein the first transmitter saddle coil and the second transmitter saddle coil are disposed axially between the transmitter axial coil and the second axial coil.

Aspect 13: Aspects of the present disclosure provide a method for measuring resistivity of reservoir fluids within a formation. The method including lowering a downhole tool into a formation of a wellbore, the downhole tool comprising a mandrel and a drill bit disposed at a distal end of the mandrel, lowering an antenna system configured to measure resistivity of a geological formation through a bore in the drill bit of the downhole tool, measuring a resistivity of the formation with the antenna system, and raising the downhole tool and the antenna system through the wellbore.

Aspect 14: The method of Aspect 13, wherein the antenna system includes a transmitter and a receiver.

Aspect 15: The method of Aspect 14, wherein the transmitter includes: a transmitter axial coil disposed around a transmitter body; a first transmitter saddle coil disposed on an outer surface of the transmitter body including a central axis perpendicular to a central axis of the transmitter axial coil; and a second transmitter saddle coil disposed on the outer surface of the transmitter body including a central axis perpendicular to the central axis of the transmitter axial coil and perpendicular to the central axis of the first transmitter saddle coil.

Aspect 16: The method of Aspect 15, wherein the transmitter body includes a magnetic core.

Aspect 17: The method of any of Aspects 14-16, wherein the receiver includes: a receiver axial coil disposed around a receiver body; a first receiver saddle coil disposed on an outer surface of the receiver body including a central axis perpendicular to the central axis of the axial receiver coil; and a second receiver saddle coil disposed on the outer surface of the receiver body including a central axis perpendicular to the central axis of the receiver axial coil and perpendicular to the central axis of the first receiver saddle coil.

Aspect 18: The method of any of Aspects 14-17, wherein the antenna system further includes a second transmitter.

Aspect 19: The method of any of Aspects 13-18, wherein measuring the resistivity of the formation with the antenna system comprises measuring the resistivity of the formation in three dimensions.

Aspect 20: The method of any of Aspects 13-19, wherein the antenna system is lowered into the wellbore from about 80 feet to about 100 feet.

Any one or more components of the antenna system 210 and tool string 106 may be integrally formed together, directly coupled together, and/or indirectly coupled together and are not limited to the specific arrangement of components illustrated in Figures 1-4. Any one or more of the embodiments of the antenna system 210 and tool string 106 may be combined in whole or part with any one or more of the embodiments of the antenna system 210 and tool string 106.

The methods disclosed herein comprise one or more actions for achieving the methods. The method actions may be interchanged with one another without departing from the scope of the claims. In other words, unless a specific order of actions is specified, the order and/or use of specific actions may be modified without departing from the scope of the claims. Further, the various operations of methods described above may be performed by any suitable means capable of performing the corresponding functions.

As used herein, the term "determining" encompasses a wide variety of actions. For example, "determining" may include calculating, computing, processing, deriving, investigating, looking up (e.g., looking up in a table, a database or another data structure), ascertaining and the like. Also, "determining" may include receiving (e.g., receiving information), accessing (e.g., accessing data in a memory) and the like. Also, "determining" may include resolving, selecting, choosing, establishing and the like.

While the present disclosure has been described with respect to a number of embodiments and examples, those skilled in the art, having benefit of this disclosure, will appreciate that other embodiments can be devised which do not depart from the scope and spirit of the present disclosure.

The preceding description, for purposes of explanation, uses specific nomenclature to provide a thorough understanding of the disclosure and is provided to enable any person skilled in the art to practice the various aspects described herein. However, it will be apparent to one skilled in the art which the specific details are not required in order to practice the systems and methods described herein. The examples discussed herein are not limiting of the scope, applicability, or aspects set forth in the claims. They are not intended to be exhaustive of or to limit this disclosure to the precise forms described. Various modifications to these aspects will be readily apparent to those skilled in the art, and the general principles defined herein may be applied to other aspects. For example, changes may be made in the function and arrangement of elements discussed without departing from the scope of the disclosure. Various examples may omit, substitute, or add various procedures or components as appropriate. The examples are shown and described in order to best explain the principles of this disclosure and practical applications, to thereby enable others skilled in the art to best utilize this disclosure and various examples with various modifications as are suited to the particular use contemplated. For instance, the methods described may be performed in an order different from that described, and various actions may be added, omitted, or combined. Also, features described with respect to some examples may be combined in some other examples. For example, an apparatus may be implemented or a method may be practiced using any number of the aspects set forth herein. In addition, the scope of the disclosure is intended to cover such an apparatus or method that is practiced using other structure, functionality, or structure and functionality in addition to, or other than, the various aspects of the disclosure set forth herein. It should be understood that any aspect of the disclosure disclosed herein may be embodied by one or more elements of a claim. It is intended which the scope of this disclosure be defined by the claims and their equivalents below.

## Claims

1. An antenna system for measuring resistivity of a geological formation, comprising:
a transmitter, including:
a transmitter axial coil disposed around a transmitter body;
a first transmitter saddle coil disposed on an outer surface of the transmitter body including a central axis perpendicular to a central axis of the transmitter axial coil; and
a second transmitter saddle coil disposed on the outer surface of the transmitter body including a central axis perpendicular to the central axis of the transmitter axial coil and perpendicular to the central axis of the first transmitter saddle coil; and
a receiver, including:
a receiver axial coil disposed around a receiver body;
a first receiver saddle coil disposed on an outer surface of the receiver body including a central axis perpendicular to the central axis of the axial receiver coil; and
a second receiver saddle coil disposed on the outer surface of the receiver body including a central axis perpendicular to the central axis of the receiver axial coil and perpendicular to the central axis of the first receiver saddle coil.

2. The antenna system of claim 1, further comprising a second transmitter, wherein the transmitter is a high frequency transmitter and the second transmitter is a low frequency transmitter.

3. The antenna system of claim 1 or 2, wherein the antenna system is lowered into the wellbore from about 80 feet to about 100 feet and the receiver further includes:
a third receiver saddle coil disposed on the outer surface of the receiver body, wherein the third receiver saddle coil includes a central axis parallel to the central axis of the first receiver saddle coil, and wherein the first receiver saddle coil and the third receiver saddle coil operate in series; and
a fourth receiver saddle coil disposed on the outer surface of the receiver body, wherein the fourth receiver saddle coil includes a central axis parallel to the central axis of the second receiver saddle coil, and wherein the second receiver saddle coil and the fourth receiver saddle coil operate in series.

4. A transmitter for measuring resistivity of a geological formation, comprising:
a transmitter axial coil disposed around a transmitter body, the transmitter body including a magnetic core;
a first transmitter saddle coil disposed on an outer surface of the transmitter body including a central axis perpendicular to a central axis of the transmitter axial coil; and
a second transmitter saddle coil disposed on the outer surface of the transmitter body including a central axis perpendicular to the central axis of the transmitter axial coil and perpendicular to the central axis of the first transmitter saddle coil.

5. The transmitter of claim 4, further comprising a second axial coil, wherein the first transmitter saddle coil and the second transmitter saddle coil are disposed axially between the transmitter axial coil and the second axial coil.

6. A method for measuring resistivity of reservoir fluids within a formation, comprising:
lowering a downhole tool into a formation of a wellbore, the downhole tool comprising a mandrel and a drill bit disposed at a distal end of the mandrel;
lowering an antenna system configured to measure resistivity of a geological formation through a bore in the drill bit of the downhole tool;
measuring a resistivity of the formation with the antenna system; and
raising the downhole tool and the antenna system through the wellbore.

7. The method of claim 6, wherein the transmitter includes:
a transmitter axial coil disposed around a transmitter body;
a first transmitter saddle coil disposed on an outer surface of the transmitter body including a central axis perpendicular to a central axis of the transmitter axial coil; and
a second transmitter saddle coil disposed on the outer surface of the transmitter body including a central axis perpendicular to the central axis of the transmitter axial coil and perpendicular to the central axis of the first transmitter saddle coil.

8. The method of claim 7, wherein the transmitter body includes a magnetic core.

9. The method of claim 6, 7 or 8, wherein the receiver includes:
a receiver axial coil disposed around a receiver body;
a first receiver saddle coil disposed on an outer surface of the receiver body including a central axis perpendicular to the central axis of the axial receiver coil; and
a second receiver saddle coil disposed on the outer surface of the receiver body including a central axis perpendicular to the central axis of the receiver axial coil and perpendicular to the central axis of the first receiver saddle coil.

10. The method of any one of the claims 6 - 9, wherein measuring the resistivity of the formation with the antenna system comprises measuring the resistivity of the formation in three dimensions.
